# EUROPEAN PATENT APPLICATION

(11) **EP 2 098 252 A2**
(43) Date of publication of application: **09.09.2009**
(21) Application number: 09161096.4
(22) Date of filing: 07.06.2006
(51) Int. Cl.: A61L 9/12, A61L 9/02, A61L 9/03, A61L 9/04, A61L 9/015, A61L 9/14, A01M 1/20, A47K 5/12, B05B 11/00, B65D 81/36, B67D 5/33, G07C 9/00

(54) **Apparatus including a selective interface system between two sub-components**

(30) Priority: 09.06.2005 US 149009
(62) Divisional of application: 06772472.4
(71) Applicant: S.C.JOHNSON & SON, INC., Racine, WI 53403-2236 (US)
(72) Inventor: Cronin, John E., Essex Junction, VT 05452 (US); Cronin, Seth M., Milton, VT 05468 (US)
(74) Representative: Ruschke, Hans Edvard

(57) **Abstract**

A three-dimensional selectively actuating interface system includes mating complementary raised (30) and recessed (40) surfaces, which may be projected in the shape of an indicium, such as a trade logo or trade mark, to limit the number of spatial relationships at which two sub-parts of an assembly may be assembled. Additional complementary opposing surface irregularities (42,44,46,48) disposed on the raised and recessed surfaces provide a further mechanical restriction in a height direction on the number of possible spatial relationships at which the two sub-parts may be matably assembled. Combined, there is only a single spatial orientation at which the two sub-parts may be assembled. In addition, an electronic switching or detection system (106) may be implemented, which is activated by the proper mating of the two sub-parts. The detection system (106) acts an electronic switch for turning on (or off) another system coupled to the assembly.

## Description

### CROSS REFERENCE TO RELATED APPLICATIONS

Not applicable

### REFERENCE REGARDING FEDERALLY SPONSORED RESEARCH OR DEVELOPMENT

Not applicable

### SEQUENTIAL LISTING

Not applicable

### BACKGROUND OF THE INVENTION

### 1. Field of the Invention

The present invention relates generally to a system for selective interface between two matable sub-components of an assembly.

### 2. Description of the Background of the Invention

Systems for selectively allowing an actuating interface between two or more sub-parts of a competed assembly are well known. For example, a lock and key system, such as for a door, is designed to allow activation or deactivation of the lock only when the correct key is properly interfaced with the lock. Such lock and key systems have been adapted and improved upon for centuries.

In another form, a selective actuation interface system has been used with two part dispensing systems, which include a reusable dispensing base and replaceable product container, to allow only selected containers to be used with the dispensing base in order to prevent unauthorized refill containers from being used with the dispensing base. While many selective actuation interface systems are possible, it has sometimes been desirable to incorporate a logo or trademark as part of the interface system for product identification purposes and for aesthetic purposes.

In one instance, a refillable toner cartridge uses a cap as a mechanical key to lock the toner cartridge onto a print cartridge receptacle. The mechanical key and lock incorporate complementary logo or trademark indicia to align and releasably engage the toner cartridge and print cartridge receptacle. In another instance, a keying mechanism between a toner cartridge and a print cartridge receptacle has a plurality of openings that form visual indicia in the form of a logo or trademark that cooperate with a plurality of protrusions to temporarily lock the toner cartridge to the print cartridge receptacle. In yet a further instance, a keying mechanism for a toner cartridge and a print cartridge includes an electronic keying system having interface circuit contacts arranged in the shape of a logo or trademark.

### SUMMARY OF THE INVENTION

According to one aspect of the invention, an apparatus includes a first component that mates to a second component. The first component includes a projected surface having a shape of an indicium, and the second component has a recessed surface complementary to the projected surface and adapted to receive the projected surface therein. A first surface irregularity is disposed on the projected surface, and a second surface irregularity is disposed on the recessed surface and is adapted to interfittingly receive the first surface irregularity. The first component mates to the second component in a pre-selected spatial orientation complementary to the shape of the indicium.

According to another aspect of the invention, an apparatus for dispensing a flowable substance contained therein includes a base unit releasably attachable to a container unit containing the flowable substance. The base unit is adapted to broadcast the flowable substance into the atmosphere. One of the base unit and the container unit includes a raised surface defining a shape of a trade indicium, and the other of the base unit and the container unit includes a recessed surface that selectively engages the raised surface. The apparatus has an assembled position in which the base unit is in fluid communication with the container unit when the raised surface is selectively engaged with the recessed surface. A dispensing activator having a first portion associated with the base unit and a second portion associated with the container unit is activated to broadcast the flowable substance when the apparatus is in the assembled position.

According to a further aspect of the invention, a refill unit for releasable attachment to a base unit includes a container having an outer wall surface, a first guide member surface having a shape of a trade indicium disposed on and displaced from the outer wall surface, and a second guide member surface disposed on and displaced from the first guide member surface. The refill unit is adapted for fluid communication with the base unit when the first guide member surface and the second guide member surface are interfittingly mated with complementary second and third base unit guide member surfaces.

Other aspects and advantages of the present invention will become apparent upon consideration of the following detailed description in which;

### BRIEF DESCRIPTION OF THE DRAWINGS

FIG. 1 is an isometric view of a selective actuation interface system between a dispensing base unit and a container unit according to one aspect of the present invention;

FIG. 2 is an enlarged isometric view of a container lid shown in FIG. 1 having a male mating surface;

FIG. 3 is an enlarged isometric detail view of a portion of the male mating surface shown in FIG. 2;

FIG. 4 is an enlarged isometric view of a container lid having a male mating surface portion of a selective activation interface system according to another aspect of the present invention including a selective activation switch;

FIG. 5 is an isometric view of a base unit having a female mating surface and a selective activation switch for actuating interface with the male mating surface shown in FIG. 4;

FIGS. 6A and 6B are schematic diagrams of a selective activation switch usable with the selective activation interface system shown in FIGS. 4 and 5;

FIGS. 7A and 7B, 8A and 8B, and 9A and 9B are schematic diagrams similar to FIGS. 6A and 6B of other selective activation switches usable with the selective activation interface system shown in FIGS. 4 and 5; '

FIG. 10 is a schematic diagram of an exemplary control circuit for actuating the selective activation switches shown in FIGS. 6A-7B; and

FIG. 11 is a logic flow diagram of a control program for the control circuit shown in FIG. 10.

### DETAILED DESCRIPTION

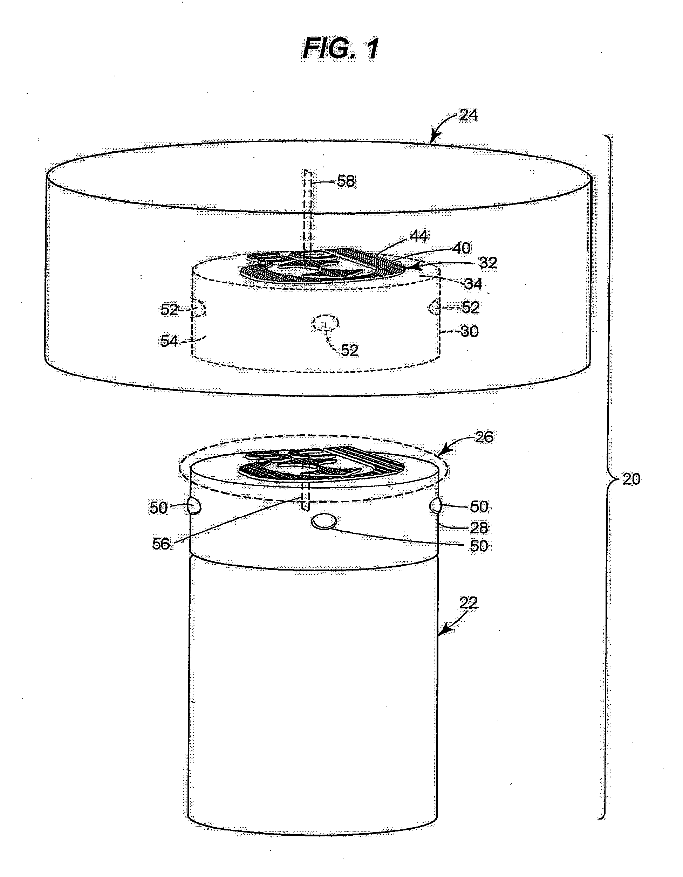

Turning now to FIGS. 1-3, in FIG. 1 an apparatus having a selectively actuating three-dimensional interface system includes two component portions that may be assembled to form complete apparatus only if complementary portions, or keys or guides, of the interface system mate to each other in a pre-selected spatial relationship. In one embodiment, the apparatus is an air care product dispenser 20 having a replaceable or refillable container unit 22 for carrying a consumable flowable or fluid substance, such as a volatile fragrance or insect control product (not shown). The container unit 22 is releasably attachable in fluid communication with a durable, or reusable dispenser base unit 24 for broadcasting or dispensing the air care product into the surrounding atmosphere. The container unit 22 has a first key, or guide portion, such as male portion 26, of the interface system disposed on a lid 28 having an outline that is snugly received within a complementary recess 30 in the dispenser base unit 24. The dispenser base unit 24 has a second key, or guide portion, such as female portion 32, of the interface system disposed on an upper wall 34 of the recess 30 that selectively engages the male portion 26 to form a substantially continuous contact or interface surface between the male and female portions of the interface system when the base unit 24 and the container unit 22 are assembled. In another embodiment, the male portion 26 is disposed on the upper wall 34 of the recess 30 and the female portion 32 is disposed on the lid 28, or each portion 26 and 32 includes both a male portion and a female portion of the interface system that are complementary with each other. Further, in yet another embodiment the male portion 26 (or the female portion 32) of the interface system is disposed on a container unit similar to the container unit 22 and not having a separable lid.

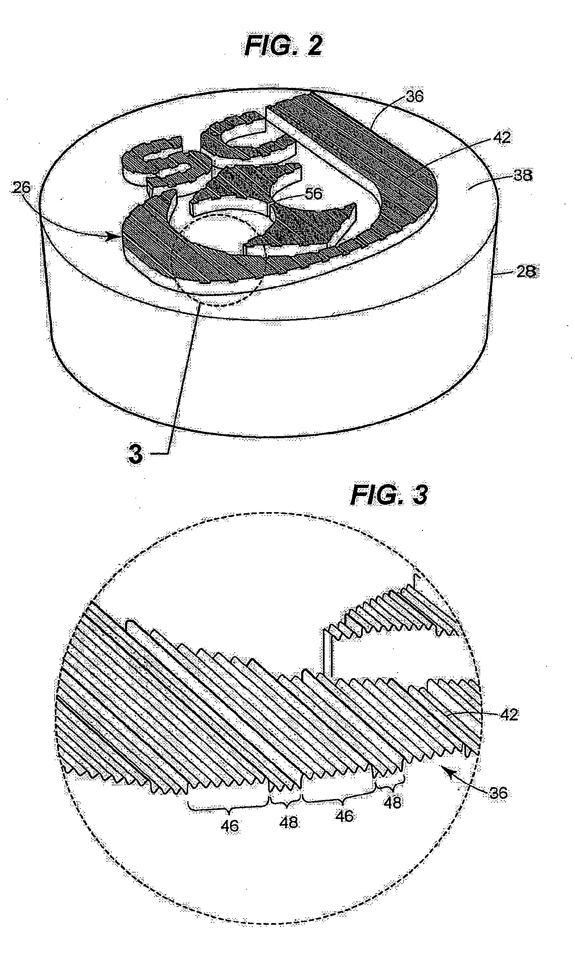

As seen more clearly in FIG. 2, the male portion 26 of the interface system includes a raised surface 36 projecting outwardly from an end wall 38 of the lid 28 and having the shape of an indicium, such as a trade logo. The female portion 32 includes a recessed surface 40 (shown in FIG. 1) projecting from the upper wall 34 that has a substantially complementary shape to the raised surface 36 such that the raised surface fits into the recessed surface at only a discrete number of possible spatial relationships. The raised surface 36 may have any desired shape including, for example, a logo, a geometric shape, a letter, a number, a random outline, etc., so that in another embodiment, the indicium has the shape of a trademark, company name, product name, slogan, picture, or another shape, which is indicatory of the nature, use, or origin of the apparatus. In another embodiment, the shape of the indicium represents other visual information such as, for example, how to assemble the components or other instructions or information. The shape of the raised surface 36 and the recessed surface 40 may be asymmetrical, or otherwise sufficiently directional, as exemplified in FIG. 2, so that the raised surface 36 selectively engages the female portion 32 at a single spatial relationship. In another embodiment, the shape is symmetrical so that the raised surface 36 selectively engages the female portion 32 at one of a plurality of possible spatial relationships.

Disposed on the raised surface 36 are surface irregularities, such as ridges 42, that interfit with complementary opposing surface irregularities, such as grooves 44 (shown in FIG. 1), disposed on the recessed surface 40, in only a discrete number of possible spatial relationships. In the case of the ridges 42 and grooves 44, the opposing surface irregularities can interfittingly mate together in only one of two possible spatial relationships. In another embodiment, a substantially random or asymmetrical pattern of surface irregularities, including, for example, bumps, dimples, protrusions, recesses, and/or other protuberances, interfit in only a single two-dimensional spatial relationship. The combination of the shape of the raised and recessed surfaces 36 and 40 and the orientation of the surface irregularities thereon, such as the ridges 42 and grooves 44, combined, allow the male portion 26 and the female portion 32 of the interface system to be matingly assembled together in only a single spatial relationship. When matingly assembled, the opposing male portion 26 and female portion 32 interfit at one or more locations and in one or more different planes, which creates an entire continuous interface surface, a plurality of discontinuous interface surface regions, and/or a plurality of discontinuous contact points, of which some are in different planes or relative heights. In one embodiment, the raised surface 36 mates with the recessed surface 40 with a substantially continuous interface surface therebetween and with the end wall 38 contacting the upper wall 34. In another embodiment, portions of the raised surface 36 and/or the recessed surface 40 are cut away such that only selected regions between the male portion 26 and the female portion 32 define a continuous interface therebetween. In yet another embodiment, the raised surface 36 mates to the recessed surface 40 such that the end wall 38 does not contact the upper wall 34 at all, or only contacts the upper wall in one area or a plurality of discrete areas. In a further embodiment, the surface irregularities on the raised surface 36 interfit with the opposing surface irregularities on the recessed surface 40 without having any other contact points between the raised surface and the recessed surface or the end wall 38 and the upper wall 34. Another embodiment includes a selectively actuating three-dimensional interface system having surface irregularities disposed on the end wall 38 that interfit with opposing surface irregularities on the upper wall 34 and having a smooth raised surface 36 opposing a smooth recessed surface 40.

As shown more clearly in FIG. 3, the ridges 42 also have a plurality of surface levels defining even further high regions 46 and low regions 48. Complementary high and low regions of the grooves 44 provide a mechanism to control the spatial relationship between the male portion 26 and the female portion 32 of the interface system in a third dimension, or height dimension, i.e., the projection dimension of the raised and recessed surfaces 36 and 40, in addition to the two-dimensional spatial restriction provided by the shape of the raised and recessed surfaces and the surface irregularities. The high and low regions 46 and 48 may be formed by surface irregularities, such as the ridges 42, of differing height from a single planar surface or by having ridges of a same height projecting from high and low regions in the raised surface 36 (and recessed surface 40). The ridges 42 having high and low regions 46 and 48 prevent a similarly shaped raised male portion 26 that does not have the correct pattern of ridges and high and low regions thereof from matingly interfacing with the female portion 32. When disassembled, such as when the male portion 26 is not matingly interfaced with the female portion 32, there is no substantially continuous interface surface (or plurality of contact points or regions located in different planes) between the male portion and the female portion; but rather, only portions of the tops of some of the ridges 42 contact the recessed surface 40 leaving a space or gap between the raised surface 36 and the recessed surface 40 at least as high as the height of the ridges.

When assembled with the male portion 26 and the female portion 32 correctly aligned and mated, a releasable latching mechanism, such as a snap fit mechanism, releasably secures the container unit 22 with the base unit 24 such that a duct or opening 56 from the container unit 22 is aligned with a duct or opening 58 in the base unit 24 to allow direct fluid communication between the base unit and the container unit. In the depicted embodiment, the snap fit mechanism includes male protrusions 50 on the side of the cap 28 that snap fit into female undercut regions 52 of a sidewall 54 of the recess 34. In other embodiments, other latching mechanism shapes are used and other latching mechanisms are used to secure the container unit 22 with the base unit 24, such as, for example, complementary magnets disposed at one or both of the cap 28 and the recess 30. In a further embodiment, the ducts 56 and 58 are offset from a center point of the lid 28 so that the ducts align with each other only when the male portion 26 and the female portion 32 of the interface system are correctly aligned. In yet another embodiment, the latching mechanisms are also selectively aligned so that the container unit 22 can only latch to the base unit 24 when the male portion 26 and the female portion 32 of the interface system are correctly aligned and mated. In a still further embodiment, the ducts 56 and 58 are configured at the raised surface 36 and the recessed surface 40 such that the duct openings directly engage each other when the ridges 42 and grooves 44 are interfitted. Otherwise, when the ducts 56 and 58 are not directly engaged, fluid flow between the base unit 24 and the container unit 22 is prevented or hindered due to the space between the raised surface 36 and the recessed surface 40 because there is no continuous path of fluid communication between the container unit and the base unit 24, which thereby prevents or otherwise hinders efficient fluid flow therebetween.

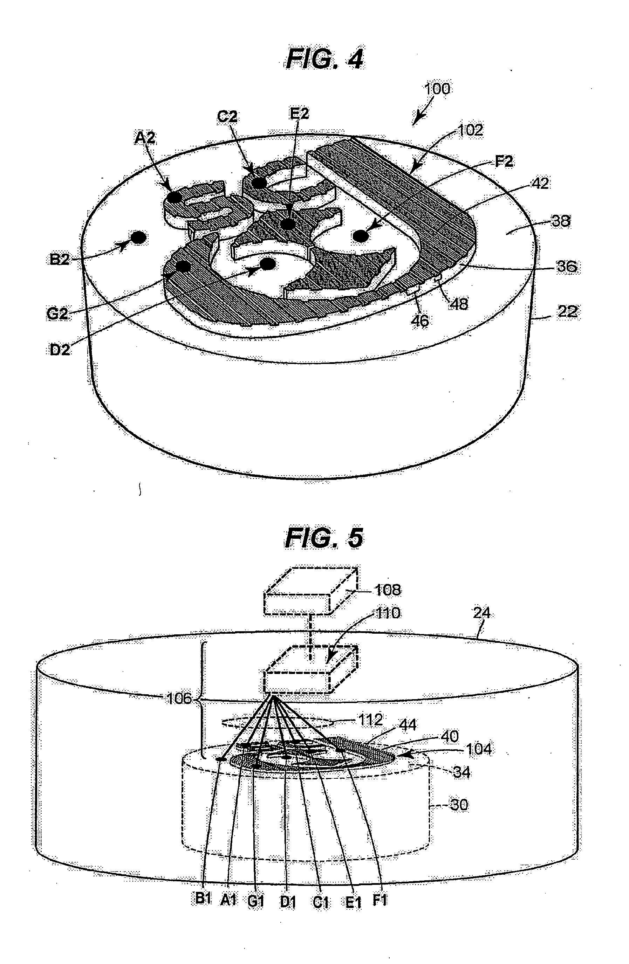

Turning now to FIGS. 4-10, in FIGS 4 and 5, another selectively actuating interface system 100, which is similar to the interface system shown in FIGS. 1-3, includes a male key portion 102 similar to the male portion 26 that selectively engages a female key portion 104 similar to the female portion 32. The male key portion 102 includes a raised surface 36 projecting a shape of a indicium, such as a trade logo, from an end wall 38 and ridges 42 having high regions 46 and low regions 48. The female key portion 104 includes a recessed surface 40 in an upper wall 34 having grooves 44 complementary to the male key portion 102 to establish a single assembled position in which an interface surface between the male key portion and the female key portion is substantially continuous or has substantially continuous regions. In another embodiment, the raised surface 36 and the recessed surface 40 mate together at a plurality of spaced apart contact regions disposed on complementary portions of the raised surface and the recessed surface, and in yet another embodiment, one or more spaced apart contacts regions are also disposed between the end wall 38 and the upper wall 34 when the ridges 42 interfit with the grooves 44. The male key portion 102 is disposed on a sub-component of a multi-component apparatus and the female key portion 104 is disposed on another sub-component of the multi-component apparatus that mates to the first component. For example, in the depicted embodiment, the male key portion 10 is disposed on a replaceable container unit 22 and the female key portion 104 is disposed on a durable base unit 24 to which the container unit releasably interlocks to provide fluid communication therebetween when the male and female key portions are mated to each other. Of course, the selectively actuating interface system may be used with other specific types of sub-component structures that matingly interface together.

In addition to the physical interface structures already described herein, the interface system 100 also includes an activation system that activates only when the male key portion 102 is correctly mated to the female key portion 104. The activation system depicted in the drawings includes a dispensing activator having a sensor with a first portion associated with the male key portion 102 and a second portion associated with the female key portion 104, such as a detection system 106, and an actuator associated with the base unit 24, such as a dispenser system 108. The detection system 106 senses the presence of a correct pre-selected mating key portion and activates or switches on the dispenser system 108 when the male key portion 102 is mated to the female key portion 104. In other embodiments, the detection system 106 acts as a switch for other types of actuators as appropriate for a particular application of the apparatus. In one embodiment, the detection system 106 includes a plurality of electrical contacts A2, B2, C2, D2, E2, F2, and G2 disposed on and around the male key portion 102. The electrical contacts A2, C2, E2, and G2 are disposed on the raised surface 36, and the electrical contacts B2, D2, and F2 are disposed on the end wall 38 near the raised surface. A complementary plurality of electrical contacts A1, B1, C1, D1, E1, F1, and G1 is disposed on and about the recessed surface 40 and the upper wall 34. Each electrical contact A1-G1 aligns with and interfaces with one corresponding electrical contact A2-G2, respectively, only when the raised surface 36 is mated to the recessed surface 40 such that the grooves 44 interfit with the ridges 42. The electrical contacts A2-G2 are complementary to the electrical contacts A1-G1, such as, for example, having female connectors disposed opposite male connectors. In one embodiment, the electrical contacts A2-G2 are male connectors and the electrical contacts A1-G1 are female connectors, but other combinations of complementary connector pairs are also possible.

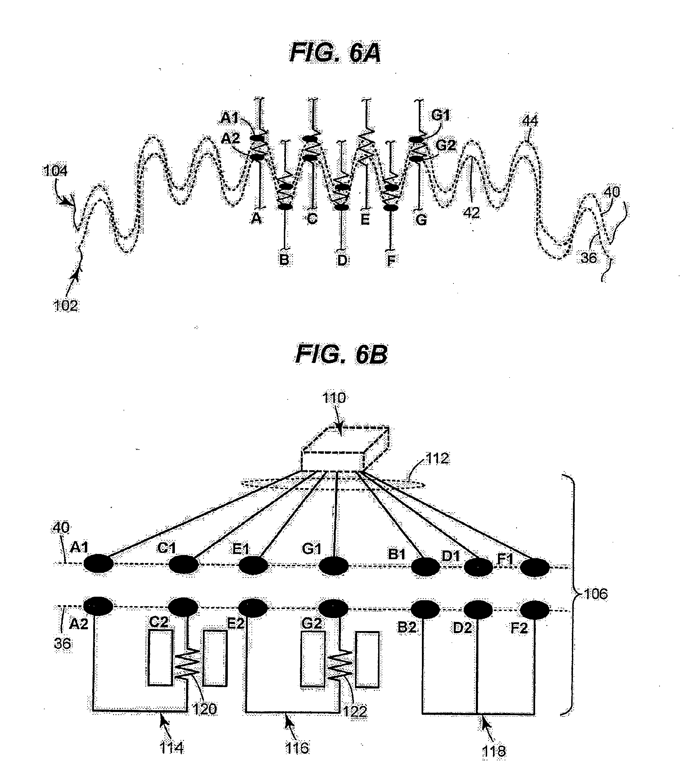

As shown diagrammatically in FIG. 6A, when the male key portion 102 is mated to the female key portion 104 in an assembled position, the contact A2 on the male key portion engages the contact A1 on the female key portion to complete an electrical connection A thereacross, and similarly with electrical contacts B2 and B1 completing electrical connection B, etc. The electrical contacts A2, C2, E2, and G2, and the corresponding electrical contacts A1, C1, E1, and, G1, are disposed at different heights or levels in the ridges 42 and grooves 44 on the respective raised surface 36 and recessed surface 40 (diagrammatically shown in each of FIGS. 6A, 7A, 8A, and 9A) such that opposing pairs of the electrical contacts can only touch if the male key portion 102 is correctly mated to the female key portion 104. In another embodiment, the electrical contacts A2, C2, E2, and G2, and the corresponding electrical contacts A1, C1, E1, and, G1, are disposed at a common height or level in the ridges 42 and grooves 44. The detection system 106 is configured such that the dispenser system 108 is actuated when all of the electrical connections A-F are simultaneously formed, which occurs when the correct male key portion 102 is mated to the female key portion 104. In this manner, if a counterfeit male key is inserted into the recess 30, the dispenser system 108 cannot be activated if any of the contacts A1-G1 are not engaged by a complementary contact A2-G2, which can occur if the shape of the raised surface 36 does not match the shape of the recessed surface 40 or if the ridges 42 are not completely mated or interfit with the grooves 44 thereby creating a space between the raised surface 36 and the recessed surface 40 due to the three-dimensional misalignment. An electrical response is generated when the electrical connections A-G are formed and detected by a detector circuit 110, coupled to the electrical contacts A1-G1 by circuit pathways 112. The dispenser system 108 is configured to operate when the detector circuit 110 receives a proper pre-selected electrical response, or validation signal, from the connections A-G, and in one embodiment, the dispenser system 108 is configured to operate only if all of the electrical connections A-G exist at the same time. In another embodiment, the detector circuit 110 includes a decoder circuit that allows activation of the dispenser system 108 when the detector circuit receives a pre-selected validation signal including a pre-selected code driven by the correct mating of the correct surfaces with correct electrical contact points mating.

In one embodiment of a detection system 106 (shown diagrammatically in FIGS. 6A and 6B), three basic circuits, signal circuits 114 and 116 and ground circuit 118, are completed by the electrical connections A-G. Signal circuit 114 is disposed between electrical contacts A2 and C2 and includes a code resistor 120, signal circuit 116 is disposed between electrical contacts E2 and G2 and includes a code resistor 122, and ground circuit 118 is disposed across electrical contacts B2, D2, and F2. The code resistors 120 and 122 are different from each other and transmit pre-selected validation signals to the detector circuit 110, and the ground circuit 118 creates a short circuit that is detectable by the detector circuit.

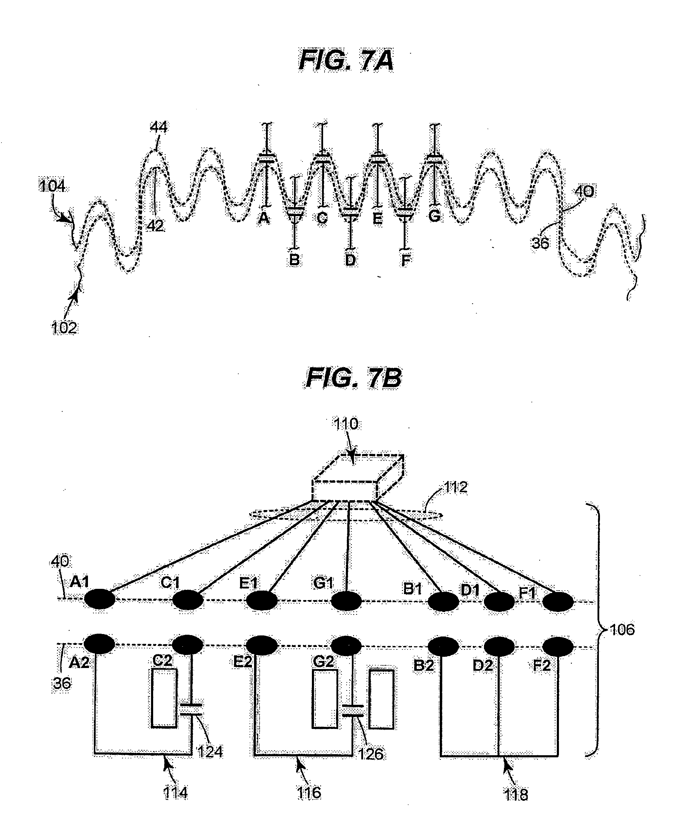

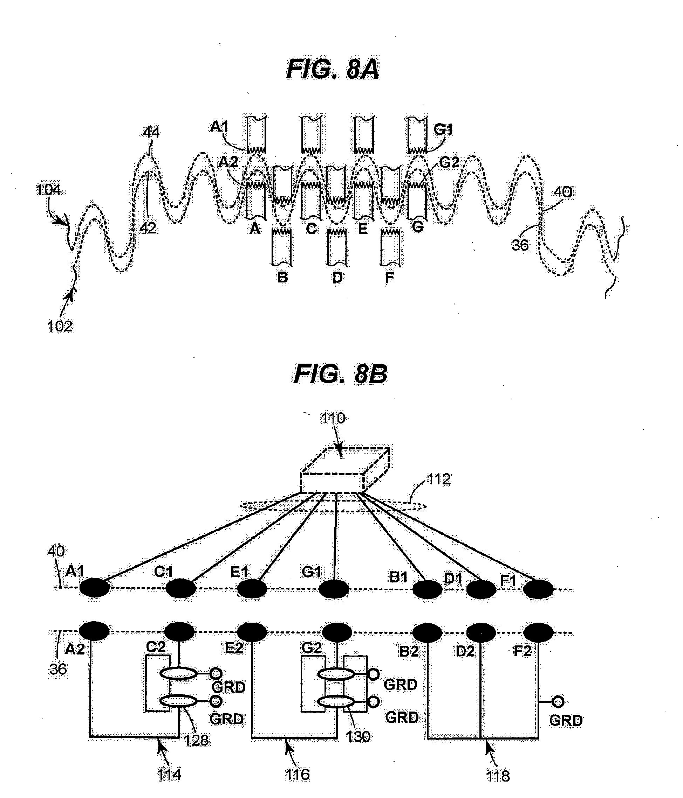

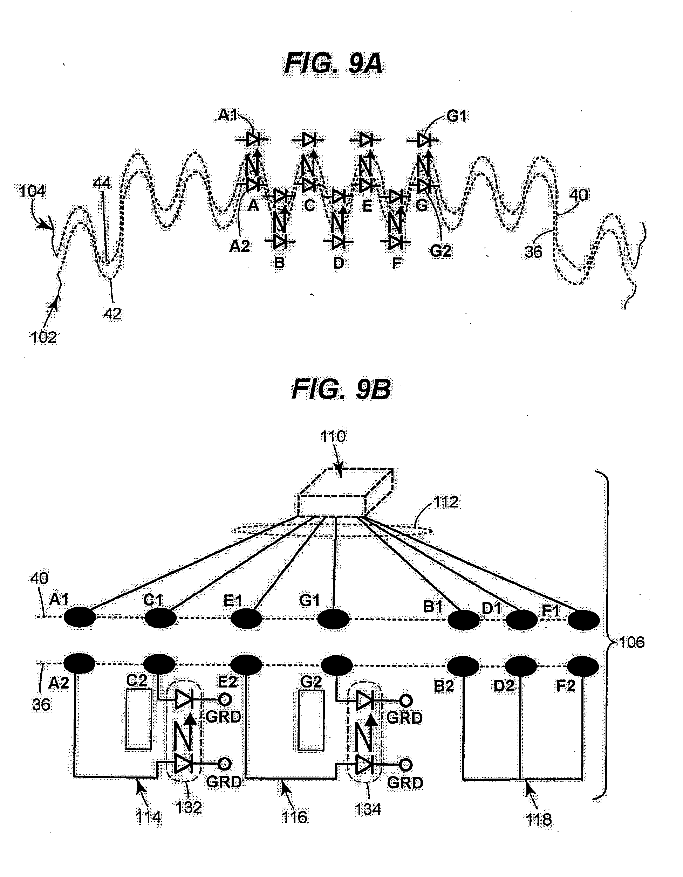

The circuits that connect to the detection system 106 may have any number and/or configuration depending on the overall configuration of the detection system as long as a pre-selected, identifiable validation signal or set of validation signals is generated to allow activation of the dispenser system 108. For example, in another embodiment of a detection system shown diagrammatically in FIGS. 7A and 7B, each of signal circuits 114 and 116 includes a different pre-selected capacitor 124 and 126, respectively, that transmits a pre-selected validation signal to a detector circuit 110, and a ground circuit 118 creates a short circuit that is detectable by the detector circuit. In yet another embodiment, shown diagrammatically in FIGS. 8A and 8B, each of signal circuits 114 and 116 includes a separate and different pre-selected inductor 128 and 130, respectively, that transmits a pre-selected validation signal to a detector circuit 110. In a further embodiment, shown diagrammatically in FIGS. 9A and 9B, each of signal circuits 114 and 116 includes a different pre-selected photo emitter and detector pair 132 and 134, respectively, that transmits a pre-selected validation signal to a detector circuit 110. In even further embodiments, the detector system may not include the ground circuit 118, or may have more or fewer signal circuits similar to any of the signal circuits described herein or have yet other devices that can generate a pre-selected, identifiable validation signal to the detection circuit 110. In yet another embodiment, the detection circuit 110 may be eliminated, and the electrical connections A-G (or any other number of appropriate electrical connections) may be coupled directly to the dispenser system 108 to complete one or more necessary circuits to allow activation of the dispenser system.

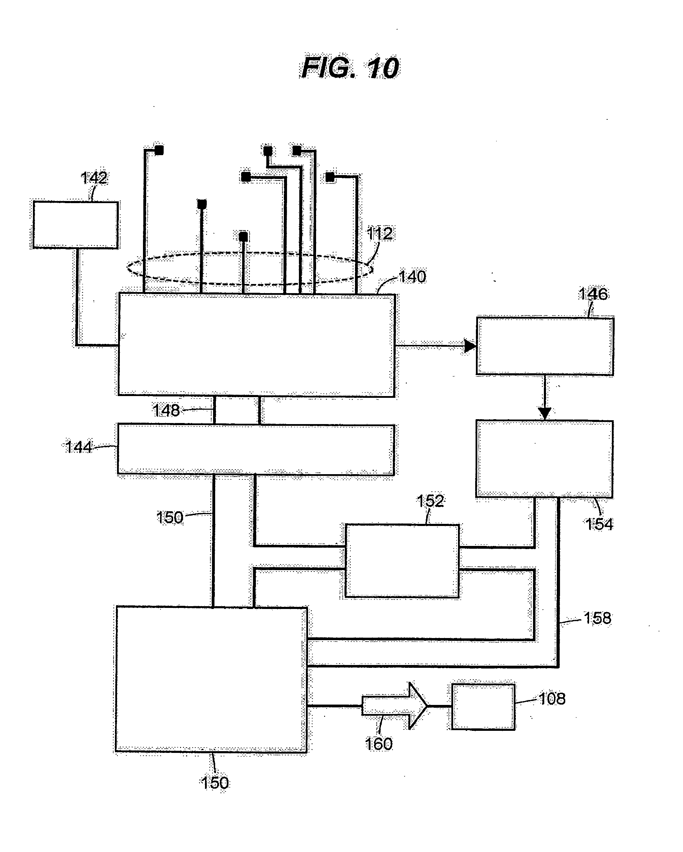

In one embodiment, a detector circuit 110, shown diagrammatically in FIG. 10, includes a programmable switch array 140 interposed between each of electrical connections A-G coupled via circuit pathways 112, a driver circuit 142, an address generator, such as address decoder 144 via an address code bus 148, and an amplifier, such as sense amplifier 146. The programmable switch array 140 selectively couples each electrical connection A-G to the driver circuit 142 in response to any one of a pre-selected plurality of address signals received from the address decoder 144. A microprocessor 150 and an electronic memory, such as memory circuit 152, are interposed in parallel between the address decoder 144 and an analog-to-digital converter (A/D converter) 154, which is coupled to the sense amplifier 146. A data bus 156 is coupled to each of the address decoder 144, memory circuit 152, and microprocessor 150; and a data bus 158 is coupled to each of the A/D converter 154, the memory circuit, and the microprocessor. The microprocessor 150 and the memory circuit 152 work in concert to transmit signals to the signal circuits 114 and 116 and the ground circuit 118 and to receive and compare the returned validation signals to determine whether the correct male key portion 102 is mated to the female key portion 104.

In operation, the memory circuit 152 directs the microprocessor 150 to transmit an address signal to the address decoder 144 via data bus 156. In response to the address signal, the address decoder 144 transmits a switching address code to the programmable switch array 140. In response to the switching address code, the programmable switch array 140 switchably couples the driver circuit 142 to one or more of the electrical connections A-G. The memory circuit 152 then directs the microprocessor 150 to transmit a second address signal to the address decoder 144, which in turn transmits a second switching address code to the programmable switch array 140, which in turn switchably couples another of the electrical connections A-G with the sense amplifier 146 to send a validation signal thereto. The sense amplifier 146 amplifies and transmits the validation signal to the A/D converter 154, which converts the validation signal into digital form and transmits the converted validation signal to the microprocessor 150. The microprocessor then compares the validation signal to a stored data value maintained in the memory circuit 152 and determines whether the validation signal matches the stored data value. This process is repeated for each of the signal circuits 114 and 116 and the ground circuit 118, and if all the validation signals are correct, then the microprocessor transmits an activation signal, or "on" signal 160, to the dispensing system 108 to activate the dispensing system.

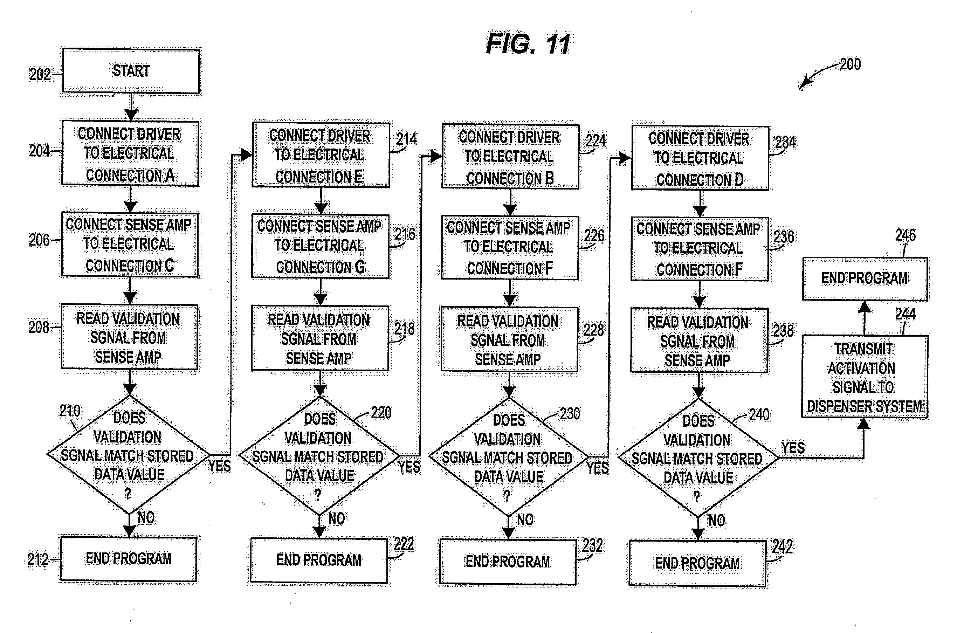

Turning now to FIG. 11, a logic diagram of one possible embodiment of a control program 200 for a detector circuit 110 coupled with a detection system 106 is started at step 202 by any sufficient initiation method, such as with an initial electrical pulse that occurs when any of the electrical connections A-G are established or by a recurring initiation pulse generated by the microprocessor 150 or the dispenser system 108. At steps 204 through 212, which form one validation loop, the control program checks for signal circuit 114. At step 204, the microprocessor 150 is directed by the memory circuit 152 to transmit an address signal that causes the programmable switch array 140 to connect the driver circuit 142 with electrical connection A. At step 206, the microprocessor 150 is directed by the memory circuit 152 to transmit a second address signal that causes the programmable switch array 140 to connect the sense amplifier 146 with electrical connection C. At step 208, the microprocessor 150 reads the validation signal returned from the signal circuit 114, and at step 210, compares the validation signal to a stored data value. If the validation signal and the stored data value do not match, then the program aborts at step 212. If the validation signal and the stored data value match, then the program continues to a next validation loop at step 214. Similar validation loops are repeated for signal circuit 116 at steps 214-222, coupling electrical connections E and G, and ground circuit 118, sequentially coupling electrical connections B and F at steps 224-232 and then electrical connections D and F at steps 234-242. If the program is still running after step 240, then the processor 150 transmits the "on" signal 160 at step 244 and ends the program at step 246, thereby activating the dispenser system 108. In another embodiment, the control program 200 validates each signal circuit 114, 116 and ground circuit 118 in another order or simultaneously. In yet another embodiment, the detection system includes more or fewer signal circuits and/or ground circuits, and the control program 200 includes additional or fewer validation loops. In a further embodiment, the control program 200 returns to step 202 or another validation loop after transmitting the activation signal at step 244 to continually re-validate and re-transmit the activation signal 160 such that, for example, a base unit 24 automatically turns off when a container unit 22 is not mated within the recess 30 or a product level detection signal is not received.

### INDUSTRIAL APPLICABILITY

The present invention may be used with a dispensing apparatus for an air care product having a non-consumable dispensing base unit that connects to a consumable container for the air care product. The dispensing unit may include a dispensing system such as a wick and a fan, a tympanic resonator, a thermal evaporator, or other known dispensing systems for broadcasting an air care product into the atmosphere. The consumable container may be re-usable, single use, or a refill container filled with air care product, such as a volatile fragrance, insect repellent or insecticide, sanitizer, and/or other similar product in a form, such as liquid, gaseous, or granular, that allows fluid flow between the container and the dispenser. The present invention may also be used with other two-part dispensing systems, such as, for example, a single use container and a spray pump for cleaner products, or a dispenser cap and a refill container for shave products.

The present invention may further be used for two part apparatuses that are not dispensing systems. A three-dimensional interface system such as described herein may be used for almost any multi-part system to allow only certain parts to be assembled together in a mating relationship. Further, when combined with the three-dimensional interface system, an electronic detection system such as described herein may be particularly useful when any part of the multi-part system includes electronic actuation systems, such as, for example, a dispensing activator for broadcasting an air care product to the surrounding atmosphere. Selectively allowing only certain mating relationships between sub-parts of a multi-part system is useful to ensure that only the correct two sub-parts are mated together and also facilitates easily assuring a proper mating relationship between the two sub-parts.

Numerous modifications to the present invention will be apparent to those skilled in the art in view of the foregoing description. Accordingly, this description is to be construed as illustrative only and is presented for the purpose of enabling those skilled in the art to make and use the invention and to teach the best mode of carrying out same. The exclusive rights to all modifications within the scope of the impending claims are reserved. It will also be appreciated that certain embodiments of the invention provide apparatus and refill units as defined in the following numbered paragraphs:
Paragraph 1. An apparatus, comprising: a first component that mates to a second component; the first component including a projected surface having a shape of an indicium; the second component having a recessed surface complementary to the projected surface and adapted to receive the projected surface therein; a first surface irregularity disposed on the projected surface; a second surface irregularity disposed on the recessed surface and adapted to interfittingly receive the first surface irregularity; wherein the first component mates to the second component in a pre-selected spatial orientation complementary to the shape of the indicium.
Paragraph 2. The apparatus of paragraph 1, wherein the first component includes a reusable dispensing unit and the second component includes a container for a product to be dispensed by the dispensing unit.
Paragraph 3. The apparatus of paragraph 1, wherein the first component mates to the second component in at most one pre-selected spatial orientation complementary to the shape of the indicium with the first surface irregularity interfittingly received by the second surface irregularity.
Paragraph 4. The apparatus of paragraph 1, wherein the shape is a trade indicium.
Paragraph 5. The apparatus of paragraph 1, wherein the first surface irregularity includes a high region having a high point at a first level and a low region having another high point at another level.
Paragraph 6. The apparatus of paragraph 1, wherein the first surface irregularity includes a groove and the second surface irregularity includes a ridge that interfits with the groove.
Paragraph 7. The apparatus of paragraph 1, further including a first portion of a detection circuit carried by the first component and a second portion of the detection circuit carried by the second component, wherein the first portion of the detection circuit and the second portion of the detection circuit are engaged to complete the detection circuit only when the first component mates to the second component.
Paragraph 8. An apparatus for dispensing a flowable substance contained therein, the apparatus comprising: a base unit releasably attachable to a container unit containing the flowable substance, the base unit adapted to broadcast the flowable substance into the atmosphere; one of the base unit and the container unit including a raised surface defining a shape of a trade indicium and the other of the base unit and the container unit including a recessed surface that selectively engages the raised surface; wherein the apparatus has an assembled position in which the base unit is in fluid communication with the container unit when the raised surface is selectively engaged with the recessed surface; and a dispensing activator having a first portion associated with the base unit and a second portion associated with the container unit, wherein the dispensing activator is activated to broadcast the flowable substance when the apparatus is in the assembled position.
Paragraph 9. The apparatus of paragraph 8, further comprising a decoder circuit associated with the dispensing activator, wherein the decoder circuit enables the dispensing activator to activate when a pre-selected validation signal is received thereby.
Paragraph 10. The apparatus of paragraph 8, wherein the dispensing activator includes an electrical signal circuit having a first circuit portion associated with the base unit and a second circuit portion associated with the container unit, wherein the electrical signal circuit allows activation of the dispensing activator only when the apparatus is in the assembled position.
Paragraph 11. The apparatus of paragraph 10, wherein the first circuit portion includes a first electrical contact and the second circuit portion includes a second electrical contact, wherein the first electrical contact interfaces with the second electrical contact only when the apparatus is in the assembled position.
Paragraph 12. The apparatus of paragraph 8, further comprising a first surface irregularity disposed on the raised surface that is complementary to a second surface irregularity disposed on the recessed surface, wherein the first surface irregularity interfits with the second surface irregularity when the apparatus is in the assembled position.
Paragraph 13. The apparatus of paragraph 12, wherein the first surface irregularity and the second surface irregularity define a substantially continuous interface surface between the base dispenser unit and the container unit in the assembled position.
Paragraph 14. The apparatus of paragraph 12, wherein the first surface irregularity includes a plurality of ridges and the second surface irregularity includes a plurality of grooves that are complementary to the ridges.
Paragraph 15. The apparatus of paragraph 14, wherein one of the ridges has a first height and another of the ridges has a second height different than the first height.
Paragraph 16. The apparatus of paragraph 8, wherein the flowable substance includes a fluid volatile air care product.
Paragraph 17. A refill unit for releasable attachment to a base unit, the refill unit comprising: a container having an outer wall surface; a first guide member surface having a shape of a trade indicium disposed on and displaced from the outer wall surface; and a second guide member surface disposed on and displaced from the first guide member surface; wherein the refill unit is adapted for fluid communication with the base unit when the first guide member surface and the second guide member surface are interfittingly mated with complementary second and third base unit guide member surfaces.
Paragraph 18. The refill unit of paragraph 17, wherein the second guide member surface includes a plurality of ridges.
Paragraph 19. The refill unit of paragraph 18, wherein a first portion of the ridges has a first height at a first level and a second portion of the ridges has a second height at a second level.
Paragraph 20. The refill unit of paragraph 17, and further comprising a first portion of a sensor disposed on the outer wall surface and a second portion of a sensor disposed on the first guide member surface.

## Claims

1. An apparatus, comprising:
a first component that mates to a second component;
the first component including a projected surface having a shape of an indicium;
the second component having a recessed surface complementary to the projected surface and adapted to receive the projected surface therein;
a first surface irregularity disposed on the projected surface;
a second surface irregularity disposed on the recessed surface and adapted to interfittingly receive the first surface irregularity;
wherein the first component mates to the second component in a pre-selected spatial orientation complementary to the shape of the indicium; and
wherein the first surface irregularity includes a high region having a high point at a first level and a low region having another high point at another level.

2. The apparatus of claim 1, wherein the first component includes a reusable dispensing unit and the second component includes, a container for a product to be dispensed by the dispensing unit.

3. The apparatus of claim 1, wherein the first component mates to the second component in at most one pre-selected spatial orientation complementary to the shape of the indicium with the first surface irregularity interfittingly received by the second surface irregularity.

4. The apparatus of claim 1, wherein the shape is a trade indicium.

5. The apparatus of claim 1, wherein the first surface irregularity includes a groove and the second surface irregularity includes a ridge that interfits with the groove.

6. The apparatus of claim 1, further including a first portion of a detection circuit carried by the first component and a second portion of the detection circuit carried by the second component, wherein the first portion of the detection circuit and the second portion of the detection circuit are engaged to complete the detection circuit only when the first component mates to the second component.

7. A refill unit for releasable attachment to a base unit, the refill unit comprising:
a container having an outer wall surface; a first guide member surface having a shape of a trade indicium disposed on and
displaced from the outer wall surface; and
a second guide member surface disposed on and displaced from the first guide member surface;
wherein the refill unit is adapted for fluid communication with the base unit when the first guide member surface and the second guide member surface are interfittingly mated with complementary second and third base unit guide member surfaces.

8. The refill unit of claim 7, wherein the second guide member surface includes a plurality of ridges.

9. The refill unit of claim 8, wherein a first portion of the ridges has a first height at a first level and a second portion of the ridges has a second height at a second level.

10. The refill unit of claim 7, and further comprising a first portion of a sensor disposed on the outer wall surface and a second portion of a sensor disposed on the first guide member surface.
